(19) Europäisches Patentamt
European Patent Office
Office européen des brevets

(11) Veröffentlichungsnummer: **0 302 021 A2**

# (12) EUROPÄISCHE PATENTANMELDUNG

(21) Anmeldenummer: **88810508.7**

(22) Anmeldetag: **25.07.88**

(51) Int. Cl.⁴: **C 07 C 85/12**
C 07 C 93/00

(30) Priorität: **30.07.87 US 79625**

(43) Veröffentlichungstag der Anmeldung:
**01.02.89 Patentblatt 89/05**

(84) Benannte Vertragsstaaten:
**AT BE CH DE ES FR GB GR IT LI LU NL SE**

(71) Anmelder: **THE UNIVERSITY OF BRITISH COLUMBIA**
**2194 Health Sciences Mall**
**Vancouver British Columbia, V6T 1W5 (CA)**

(72) Erfinder: **Cullen, William Robert**
**Apt 307-5775 Toronto Road**
**Vancouver, B.C., V6T 1X4 (CA)**

**Fryzuk, Michael Daniel**
**Erfurterstrasse 21**
**D-6750 Kaiserslautern (DE)**

**James, Brian Robert**
**4010 Blensheim St.**
**Vancouver, B.C., V6L 2Y9 (CA)**

**Kang, Guo-Jun**
**Room 111-Bldg. 14 South West Village**
**Mankai University Tianjin (CN)**

**Kutney, James Peter**
**2327 McMullen Avenue**
**Vancouver, B.C., V6L 2E2 (CA)**

**Spogliarich, Roberto**
**Via Bonomea 219**
**I-34136 Trieste (IT)**

**Thorburn, Ian Stuart**
**3687 Vimy Crescent**
**Vancouver, B.C., V5M 4B6 (CA)**

(74) Vertreter: **Hofstetter, Marta et al**
**Patentabteilung CIBA-GEIGY AG**
**CH-4002 Basel (CH)**

(54) **Verfahren zur Herstellung von optisch aktiven sekundären Arylaminen.**

(57) Asymmetrische Hydrierung von prochiralen N-Arylketiminen zu optisch aktiven sekundären Aminen bei einer Temperatur von -40 bis 80°C, einem Wasserstoffdruck von $10^6$ bis $10^8$ Pa unter Zusatz katalytischer Mengen einer Rhodiumverbindung der Formel III oder IIIa

$$[XRhYZ] \quad (III) \quad \text{oder} \quad [XRhY]^{\oplus}A^{\ominus} \quad (IIIa),$$

worin X für zwei Olefinliganden oder einen Dienliganden steht, Y ein chirales Diphosphin, dessen sekundäre Phosphingruppen durch 2-4 C-Atome verknüpft sind und das zusammen mit dem Rh-Atom einen 5-, 6- oder 7-Ring bildet, oder Y ein chirales Diphosphinit, dessen Phosphinitgruppen über 2 C-Atome verknüpft sind und das zusammen mit dem Rh-Atom einen 7-Ring bildet, bedeutet, Z für Cl, Br oder I steht, und $A^{\ominus}$ das Anion einer Sauerstoff- oder Komplexsäure darstellt.



Bundesdruckerei Berlin

**Beschreibung**

**Verfahren zur Herstellung von optisch aktiven sekundären Arylaminen**

Die vorliegende Erfindung betrifft ein Verfahren zur Herstellung von optisch aktiven sekundären Aminen durch asymmetrische Hydrierung von prochiralen N-Arylketiminen mit chiralen Rhodiumdiphosphin- oder -diphosphinitkomplexen.

In der EP-A-O 104 375 sind chirale Diphosphinliganden beschrieben, deren Komplexe mit Metallen der Gruppe VIII des Periodensystems als Katalysatoren bei der asymmetrischen Hydrierung von α-(Acylamin)acrylsäuren verwendet werden können.

S. Vastag et al. beschreiben im J. of Molecular Catalysis, 22, S. 283 - 287 (1984) die asymmetrische Hydrierung von prochiralen N-Benzylketiminen unter Verwendung von Rhodiumkomplexen mit chiralen Diphosphinliganden. Der chemische Umsatz und die Reproduzierbarkeit der optischen Ausbeuten sind gering.

Es wurde gefunden, dass Rhodiumverbindungen mit chiralen Diphosphin- oder Diphosphinitliganden besonders geeignete homogene asymmetrische Katalysatoren für die Hydrierung von prochiralen N-Arylketiminen sind. Unter milden Reaktionsbedingungen werden bei hohen chemischen Umsätzen erhöhte und reproduzierbare optische Ausbeuten an optisch aktiven sekundären N-Arylaminen erzielt. Optisch aktiv bedeutet einen Ueberschuss eines Enantiomeren mit R- oder S-Konfiguration.

Gegenstand vorliegender Erfindung ist ein Verfahren zur Herstellung von optisch aktiven sekundären N-Arylaminen der Formel I

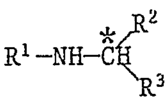 (I),

worin $R^1$ $C_6$-$C_{12}$-Aryl oder über ein Ring-C-Atom gebundenes $C_4$-$C_{11}$-Heteroaryl mit 1 oder 2 Heteroatomen im Ring bedeutet, die durch $C_1$-$C_{12}$-Alkyl, $C_1$-$C_{12}$-Alkoxy, $C_1$-$C_{12}$-Alkylthio, $C_1$-$C_6$-Haloalkyl, Halogen, -OH, -CN, $C_6$-$C_{12}$-Aryl oder -Aryloxy oder -Arylthio, $C_7$-$C_{16}$-Aralkyl oder -Aralkoxy oder -Aralkylthio, wobei die Arylreste ihrerseits durch $C_1$-$C_4$-Alkyl, -Alkoxy, -Alkylthio, Halogen, -OH, -CN, $-CONR^4R^5$ oder $-COOR^4$ substituiert sein können, Sekundäramino mit 2 bis 24 C-Atomen, $-\overset{O}{\overset{\|}{C}}-NR^4R^5$ oder $-COOR^4$, wobei $R^4$ und $R^5$ unabhängig voneinander $C_1$-$C_{12}$-Alkyl, Phenyl oder Benzyl oder $R^4$ und $R^5$ zusammen Tetra- oder Pentamethylen oder 3-Oxapentylen sind, substituiert sein können;

$R^2$ und $R^3$ voneinander verschieden sind und gegebenenfalls durch -OH, -CN, Halogen, $C_1$-$C_{12}$-Alkoxy, Phenoxy, Benzyloxy, Sekundäramino mit 2 bis 24 C-Atomen, $-\overset{O}{\overset{\|}{C}}NR^4R^5$ oder $-COOR^4$ substituiertes $C_1$-$C_{12}$-Alkyl oder Cycloalkyl mit 3-8 Ring-C-Atomen, gegebenenfalls wie $R^1$ substituiertes $C_6$-$C_{12}$-Aryl oder $C_7$-$C_{16}$-Aralkyl, $-CONR^4R^5$ oder $-COOR^4$ darstellen, worin $R^4$ und $R^5$ die zuvor angegebene Bedeutung haben; oder

$R^1$ die zuvor angegebene Bedeutung hat und $R^2$ und $R^3$ zusammen gegebenenfalls durch 1 oder 2 -O-, -S- oder $-NR^4-$ unterbrochenes, und/oder gegebenenfalls durch =O oder wie zuvor für $R^2$ und $R^3$ in der Bedeutung von Alkyl substituiertes, und/oder mit Benzol, Furan, Thiophen oder Pyrrol kondensiertes Alkylen mit 2 bis 5 C-Atomen sind, oder

$R^2$ die zuvor angegebene Bedeutung hat und $R^3$ an $R^1$ gebundenes, gegebenenfalls durch 1 oder 2 -O-, -S- oder $-NR^4-$ unterbrochenes, und/oder gegebenenfalls durch =O oder wie zuvor für $R^2$ und $R_3$ in der Bedeutung von Alkyl substituiertes, und/oder mit Benzol, Furan, Thiophen oder Pyrrol kondensiertes Alkylen mit 2 bis 5 C-Atomen sind, und * für überwiegend R- oder S-Konfiguration steht, durch asymmetrisch katalysierte Hydrierung von N-arylierten prochiralen Ketiminen der Formel II

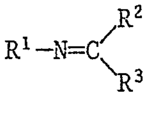 (II),

worin $R^1$, $R^2$ und $R^3$ die zuvor angegebenen Bedeutungen haben, in Gegenwart von Komplexsalzen eines Edelmetalls mit chiralen Liganden, das dadurch gekennzeichnet ist, dass man die Hydrierung bei einer Temperatur von -40 bis 80°C und einem Wasserstoffdruck von $10^6$ Pa bis $10^8$ Pa vornimmt und dem Reaktionsgemisch katalytische Mengen einer Rhodiumverbindung der Formel III oder IIIa

$= [XRhYZ]$ (III) oder $[XRhY]^{\oplus}A^{\ominus}$ (IIIa)

zugibt, worin X für zwei Olefinliganden oder einen Dienliganden steht, Y ein chirales Diphosphin, dessen sekundäre Phosphingruppen durch 2-4 C-Atome verknüpft sind und das zusammen mit dem Rh-Atom einen 5-, 6- oder 7-Ring bildet, oder Y ein chirales Diphosphinit, dessen Phosphinitgruppen über 2 C-Atome verknüpft sind und das zusammen mit dem Rh-Atom einen 7-Ring bildet, bedeutet, Z für Cl, Br oder I steht, und $A^{\ominus}$ das Anion einer Sauerstoff- oder Komplexsäure darstellt.

$R^1$ kann in beliebigen Stellungen durch gleiche oder verschiedene Reste substituiert sein, z.B. mit 1 bis 5, vorzugsweise 1 bis 3 Substituenten. Die Substitution in den beiden Orthostellungen zum N-Atom kann einen günstigen Einfluss auf die gewünschten Ausbeuten haben, wobei in diesem Fall $R^2$ vorzugsweise nicht Aryl bedeutet. Bevorzugt sind beide Orthostellungen substituiert, insbesondere mit $C_1$-$C_{12}$-Alkyl.

Geeignete Substituenten für $R^1$ sowie $R^2$ und $R^3$ in der Bedeutung von Aryl und Aralkyl sind:
$C_1$-$C_{12}$-, bevorzugt $C_1$-$C_6$- und besonders $C_1$-$C_4$-Alkyl, -Alkoxy oder -Alkylthio, z.B. Methyl, Ethyl, Propyl, n-, i- und t-Butyl, die Isomeren von Pentyl, Hexyl, Octyl, Nonyl, Decyl, Undecyl und Dodecyl, sowie entsprechende Alkoxy- und Alkylthioreste;
$C_1$-$C_6$-, vorzugsweise $C_1$-$C_4$-Haloalkyl mit vorzugsweise F und Cl als Halogen, z.B. Trifluor- oder Trichlormethyl, Difluorchlormethyl, Fluordichlormethyl, 1,1-Difluoreth-1-yl, 1,1-Dichloreth-1-yl, 1,1,1-Trichlor- oder -Trifluoreth-2-yl, Pentachlorethyl, Pentafluorethyl, 1,1,1-Trifluor-2,2-dichlorethyl, n-Perfluorpropyl, i-Perfluorpropyl, n-Perfluorbutyl, Fluor- oder Chlormethyl, Difluor- oder Dichlormethyl, 1-Fluor- oder -Chlor-eth-2-yl oder -eth-1-yl, 1-, 2- oder 3-Fluor- oder -Chlor-prop-1-yl oder -prop-2-yl oder -prop-3-yl, 1-Fluor- oder -Chlor-but-1-yl, -but-2-yl, -but-3-yl oder -but-4-yl, 2,3-Dichlor-prop-1-yl, 1-Chlor-2-fluor-prop-3-yl, 2,3-Dichlorbut-1-yl;
Halogen, bevorzugt F und Cl;
$C_6$-$C_{12}$-Aryl, -Aryloxy oder -Arylthio, in denen Aryl bevorzugt für Naphthyl und besonders Phenyl steht,
$C_7$-$C_{16}$-Aralkyl, -Aralkoxy und -Aralkylthio, in denen der Arylrest bevorzugt Naphthyl und besonders Phenyl ist und der Alkylenrest linear oder verzweigt ist und 1 bis 10, vorzugsweise 1 bis 6 und insbesondere 1-3 C-Atome enthält, z.B. Benzyl, Naphthylmethyl, 1- oder 2-Phenyleth-1-yl oder -eth-2-yl, 1-, 2- oder 3-Phenyl-prop-1-yl, -prop-2-yl oder -prop-3-yl, besonders bevorzugt ist Benzyl;
die zuvor genannten Arylgruppen enthaltenden Reste können ihrerseits ein- oder mehrfach substituiert sein, z.B. durch $C_1$-$C_4$-Alkyl, -Alkoxy oder -Alkylthio, Halogen, -OH, -CN, -$CONR^4R^5$ oder -$COOR^4$, wobei $R^4$ und $R^5$ die angegebenen Bedeutungen haben; Beispiele sind Methyl, Ethyl, n- und i-Propyl, Butyl, entsprechende Alkoxy- und Alkylthioreste, F, Cl, Br, Dimethyl-, Methylethyl-, Diethylcarbamoyl, Methoxy-, Ethoxy-, Phenoxy- und Benzyloxycarbonyl,

Sekundäramino mit 2 bis 24, vorzugsweise 2 bis 12 und besonders 2 bis 6 C-Atomen, wobei das Sekundäramino bevorzugt 2 Alkylgruppen enthält, z.B. Dimethyl-, Methylethyl-, Diethyl-, Methylpropyl-Methyl-n-butyl-, Di-n-Propyl-, Di-n-Butyl-, Di-n-Hexylamino;
-$CONR^4R^5$ , worin $R^4$ und $R^5$ unabhängig voneinander $C_1$-$C_{12}$-, vorzugsweise $C_1$-$C_6$- und insbesondere $C_1$-$C_4$-Alkyl oder $R^4$ und $R^5$ zusammen Tetra- oder Pentamethylen oder 3-Oxapentylen sind, wobei das Alkyl linear oder verzweigt sein kann, z.B. Dimethyl-, Methylethyl-, Diethyl-, Methyl-n-propyl-, Ethyl-n-propyl-, Di-n-propyl-, Methyl-n-butyl-, Ethyl-n-butyl-, n-Propyl-n-butyl- und Di-n-butylcarbamoyl;

-$COOR^4$, worin $R^4$ $C_1$-$C_{12}$-, bevorzugt $C_1$-$C_6$-Alkyl ist, das linear oder verzweigt sein kann, z.B. Methyl, Ethyl, n- und i-Propyl, n-, i- und t-Butyl, und die Isomeren von Pentyl, Hexyl, Heptyl, Octyl, Nonyl, Decyl, Undecyl und Dodecyl.

$R^1$ als Aryl ist bevorzugt unsubstituiertes oder substituiertes Naphthyl und besonders Phenyl. $R^1$ als Heteroaryl ist bevorzugt ein 5- oder 6-gliedriger Ring mit 1 oder 2 gleichen oder verschiedenen Heteroatomen, besonders O, S oder N, das bevorzugt 4 oder 5 C-Atome enthält und das mit Benzol kondensiert sein kann. Beispiele für Heteroaromaten, von denen sich $R^1$ ableiten kann, sind Furan, Pyrrol, Thiophen, Pyridin, Pyrimidin, Indol und Chinolin.

Für die Substituenten von $R^2$ und $R^3$ gelten die gleichen Bevorzugungen wie für die Substituenten von $R^1$. $R^2$ und $R^3$ als Alkyl sind bevorzugt unsubstituiertes oder substituiertes $C_1$-$C_6$-, besonders $C_1$-$C_4$-Alkyl, das linear oder verzweigt sein kann. Beispiele sind Methyl, Ethyl, i- und n-Propyl, i-, n- und t-Butyl, die Isomeren von Pentyl, Hexyl, Heptyl, Octyl, Nonyl, Decyl, Undecyl und Dodecyl.

$R^2$ und $R^3$ als unsubstituiertes oder substituiertes Cycloalkyl enthalten bevorzugt 3 bis 6, besonders 5 oder 6 Ring-C-Atome. Beispiele sind Cyclopropyl, Cyclobutyl, Cyclopentyl, Cyclohexyl, Cycloheptyl und Cyclooctyl.

$R^2$ und $R^3$ als Aryl sind bevorzugt unsubstituiertes oder substituiertes Naphthyl und besonders Phenyl. $R^2$ und $R^3$ als Aralkyl sind bevorzugt unsubstituiertes oder substituiertes Phenylalkyl mit 1-10, bevorzugt 1 bis 6 und besonders 1 bis 4 C-Atomen im Alkylen, wobei das Alkylen linear oder verzweigt sein kann. Beispiele sind besonders Benzyl, sowie 1-Phenyleth-1-yl, 2-Phenyleth-1-yl, 1-Phenyl-prop-1-yl, 1-Phenylprop-2-yl, 1-Phenyl-prop-3-yl, 2-Phenylprop-1-yl, 2-Phenylprop-2-yl und 1-Phenylbut-4-yl.

In $R^2$ und $R^3$ als -$CONR^4R^5$ und -$COOR^4$ stellen $R^4$ und $R^5$ bevorzugt $C_1$-$C_6$-, besonders $C_1$-$C_4$-Alkyl oder $R^4$ und $R^5$ zusammen Tetramethylen, Pentamethylen oder 3-Oxapentylen dar. Beispiele für Alkyl sind zuvor erwähnt worden.

$R^2$ und $R^3$ zusammen oder an $R^1$ gebundenes $R^3$ als Alkylen sind bevorzugt durch 1 -O-, -S- oder $-NR^4-$, vorzugsweise -O-, unterbrochen. $R^2$ und $R^3$ zusammen bzw. an $R^1$ gebundenes $R^3$ bilden mit dem C-Atom bzw. mit der -N=C-Gruppe, an das sie gebunden sind, bevorzugt einen 5-oder 6-gliedrigen Ring. Für die Substituenten gelten die zuvor erwähnten Bevorzugungen. Als kondensiertes Alkylen sind $R^2$ und $R^3$ zusammen bzw. an $R^1$ gebundenes $R^3$ bevorzugt mit Benzol oder Pyridin kondensiertes Alkylen. Beispiele für Alkylen sind: Ethylen, 1,2- oder 1,3-Propylen, 1,2-, 1,3- oder 1,4-Butylen, 1,5-Pentylen und 1,6-Hexylen. Beispiele für unterbrochenes oder durch =O substituiertes Alkylen sind 2-Oxa-1,3-propylen, 2-Oxa-1,4-butylen, 2-Oxa-oder 3-Oxa-1,5-pentylen, 3-Thia-1,5-pentylen, 2-Thia-1,4butylen, 2-Thia-1,3-propylen, 2-Methylimino-1,3-propylen, 2-Ethylimino-1,4-butylen, 2- oder 3-Methylimino-1,5-pentylen, 1-Oxo-2-oxa-1,3-propylen, 1-Oxo-2-oxa-1,4-butylen, 2-Oxo-3-oxa-1,4-butylen, 1-Oxa-2-oxo-1,5-pentylen. Beispiele für kondensiertes oder an $R^1$ gebundenes Alkylen sind:

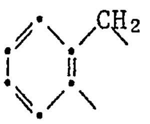 , 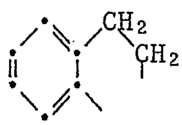 , 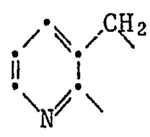 und 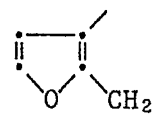 .

Beispiele für kondensiertes bzw. an $R^1$ gebundenes und unterbrochenes und gegebenenfalls mit =O substituiertes Alkylen sind

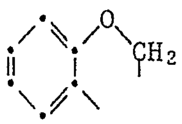 , 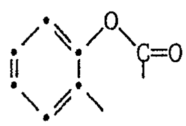 und 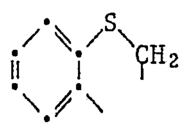 .

In einer bevorzugten Gruppe steht in Formel II $R^1$ für 2,6-Dimethylphen-1-yl oder 2-Methyl-6-ethylphen-1-yl, $R^2$ für Methyl und $R^3$ für Methoxymethyl.

N-Arylimine der Formel II sind bekannt oder sie können nach bekannten Verfahren aus Ketonen und Arylaminen hergestellt werden. In einer Ausführungsform des Verfahrens können die N-Arylimine der Formel II auch in situ aus den entsprechenden Ketonen und Arylaminen hergestellt werden.

Das Verfahren wird bevorzugt bei einer Temperatur von -30 bis 50° C, besonders -25 bis 20° C, insbesondere -25 bis 10° C, und bevorzugt bei einem Wasserstoffdruck von $4 \cdot 10^6$ bis $5 \cdot 10^7$ Pa, besonders $7 \cdot 10^6$ bis $3 \cdot 10^7$ Pa durchgeführt.

In den Formeln III und IIIa kann X als Olefinligand z.B. Buten, Propen und besonders Ethylen sein und der Dienligand ist bevorzugt ein offenkettiges oder cyclisches Dien, dessen Diengruppen durch ein oder zwei C-Atome verknüpft sind. Das Dien ist bevorzugt Hexadien, Cyclooctadien oder besonders Norbornadien.

Im chiralen Diphosphin sind die Phosphingruppen bevorzugt über eine aliphatische Gruppe mit 2-4 C-Atomen verknüpft, die durch $C_1$-$C_4$-Alkyl, $C_5$- oder $C_6$-Cycloalkyl, Phenyl oder Benzyl substituiert sein kann. Bei der aliphatischen Gruppe kann es sich um Alkylen oder um eine cycloaliphatische Gruppe mit 5 oder 6 Ring-C-Atomen oder um eine aliphatisch-heterocyclische Gruppe mit 1 bis 2 -O- oder $=N$-$C_1$-$C_{12}$-Alkyl, oder -Acyl oder -Aminocarbonyl, -Phenyl oder -Benzyl und 3-5 C-Atomen im Ring handeln. Die Ringe können durch $C_1$-$C_4$-Alkyl, $C_5$-$C_7$-Cycloalkyl, Phenyl oder Benzyl substituiert sein.

Y in den Formeln III oder IIIa ist bevorzugt ein chirales Diphosphin, dessen Phosphingruppen durch 2 C-Atome verknüpft sind und das zusammen mit dem Rh-Atom einen 5-Ring bildet.

Die Phosphingruppen und Phosphinitgruppen enthalten bevorzugt $C_1$-$C_{12}$-Alkyl, Cycloalkyl mit 5 bis 8 Ring-C-Atomen, das durch 1 bis 3 $C_1$-$C_6$-Alkylgruppen substituiert sein kann, Phenyl, $C_7$-$C_{12}$-Phenylalkyl oder Alkylphenylalkyl mit 1 bis 6 C-Atomen in den Alkylgruppen und 1 bis 5 C-Atomen in der Alkylengruppe. Besonders bevorzugt sind t-Butyl, Phenyl, Benzyl oder Cyclohexyl. Geeignete chirale Diphosphine sind beschrieben in H.B. Kagan, Chiral Ligands for Asymmetric Catalysis, Asymmetric Synthesis, Volume 5, S. 13-23, Academic Press, Inc., N.Y. (1985).

Beispiele sind (Ph steht für Phenyl):

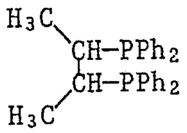 , 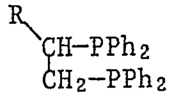 R = Methyl, Phenyl, Cyclohexyl,

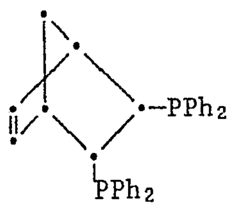 , 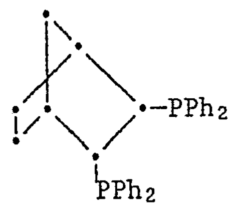 ,

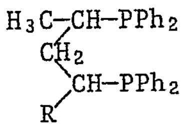 R=H,$CH_3$ , 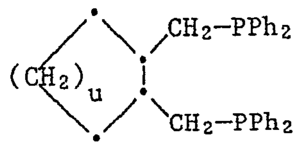 u=0, 1, 2 ,

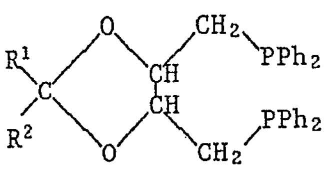 $R^1$=H,$CH_3$,Ph, $R^2$=H,$CH_3$,Ph 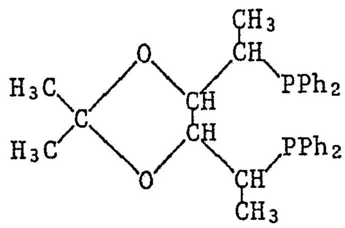 ,

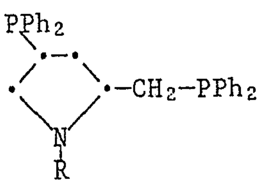 R=$-CO_2$-t-Butyl, -CO-t-Butyl, -CONH$C_1$-$C_4$-Alkyl,

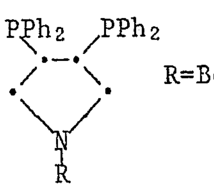 R=Benzyl, $C_1$-$C_4$-Alkyl.

Ein Beispiel für Disphosphinite ist 1-0-Phenyl-4,6-0-(R)-benzyliden-2,3-0-bis(diphenylphosphino)-β-D-glucopyranosid der Formel

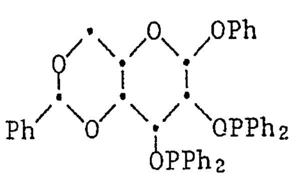 .

In Formel III steht Z bevorzugt für Cl oder Br. $A^{\ominus}$ in Formel IIIa steht bevorzugt für $ClO_4^{\ominus}$, $CF_3SO_3^{\ominus}$, $BF_4^{\ominus}$, $PF_6^{\ominus}$, $SbCl_6^{\ominus}$, $AsF_6^{\ominus}$ oder $SbF_6^{\ominus}$.

Eine bevorzugte Gruppe von Rhodiumverbindungen sind solche der Formel III, worin X für Norbornadien, Z für Cl und Y für (R)- oder (S)-

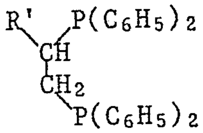

stehen, worin R′ Methyl, Phenyl oder Cyclohexyl bedeutet.

Die Rhodiumverbindungen der Formeln III und IIIa sind bekannt oder können nach bekannten Verfahren hergestellt werden, siehe z.B. M. Green et al., J. Chem. Soc. (A), S. 2334 ff (1971).

Die Rhodiumverbindungen können als isolierte Verbindungen eingesetzt werden. Es ist zweckmässig, die Verbindungen in situ herzustellen und direkt zu verwenden.

Die Rhodiumverbindungen werden bevorzugt in Mengen von 0,01 bis 5, besonders 0,05 bis 2 Mol-% eingesetzt, bezogen auf die Verbindungen der Formel II. Das Molverhältnis von Verbindung der Formel II zu Verbindung der Formel III bzw. IIIa kann 1000 bis 20, bevorzugt 200 bis 50 betragen.

Die Reaktion kann ohne oder in Gegenwart von Lösungsmitteln durchgeführt werden. Geeignete Lösungsmittel, die alleine oder als Mischung von Lösungsmitteln eingesetzt werden können, sind zum Beispiel:

Aliphatische und aromatische Kohlenwasserstoffe, wie z.B. Pentan, Hexan, Cyclohexan, Methylcyclohexan, Benzol, Toluol und Xylol; Alkohole, wie z.B. Methanol, Ethanol, Propanol und Butanol; Ether, wie z.B. Diethylether, Diethylenglykoldimethylether, Tetrahydrofuran und Dioxan; Halogenkohlenwasserstoffe, wie z.B. Methylenchlorid, Chloroform, 1,l,2,2-Tetrachlorethan und Chlorbenzol; Ester und Lactone, wie z.B. Essigsäureethylester, Butyrolacton und Valerolacton, Säureamide und Lactame, wie z.B. Dimethylformamid, Dimethylacetamid und N-Methylpyrrolidon. Bevorzugt verwendet man Mischungen von Alkoholen und aromatischen Kohlenwasserstoffen, z.B. Methanol/Benzol oder Methanol/Toluol.

Die Verbindungen der Formel I sind biologisch aktive Substanzen oder Zwischenprodukte zur Herstellung solcher Substanzen mit einer N-Arylsekundäramingruppe, insbesondere im Bereich der Pharmazeutika und Agrochemikalien. So wirken z.B. o,o-Dialkylarylketaminderivate, insbesondere solche mit Alkyl- und/oder Alkoxyalkylgruppen, als Fungizide, besonders als Herbizide. Bei den Derivaten kann es sich um Aminsalze, Säureamide, z.B. von Chloressigsäure, tertiäre Amine und Ammoniumsalze handeln (siehe z.B. EP-A-O 077 755 und EP-A-O 115 470).

Die nachfolgenden Beispiele erläutern die Erfindung näher.

Beispiel 1: In einen 250 ml Zweihalskolben wird unter $N_2$-Schutzgas N(2,6-Dimethylphen-1-yl)-methoxymethyl-methylketimin eingetragen. Der Kolben wird auf $5 \cdot 10^3$ Pa evakuiert und mit Stickstoff gespült. Danach wird das Lösungsmittel zugegeben und 2 Minuten bei Raumtemperatur gerührt (Lösung A).

In einen 50 ml Zweihalskolben wird unter $N_2$-Schutzgas das Lösungsmittel eingetragen. Danach werden nacheinander $[Rh (Norbornadien)Cl]_2$, und chirales Diphosphin zugefügt. Nach jeder Zugabe wird so lange gerührt, bis eine homogene Lösung vorliegt (Lösung B).

In einen 0,3 1 Stahlautoklaven werden unter Luftausschluss mit einer Kapillare die Lösungen A und B nacheinander eingetragen. Durch ein Gaseinlassventil werden $6{,}9 \cdot 10^6$ Pa Wasserstoff aufgepresst. Die Temperatur beträgt 20°C. Die Reaktion wird solange bei einem konstanten Wasserstoffdruck von $6{,}9 \cdot 10^6$ Pa durchgeführt, bis keine Wasserstoffaufnahme mehr erfolgt. Danach wird das Reaktionsgemisch mit Stickstoff in einen 250 ml Kolben gespült.

Das Lösungsmittel wird bei 80°C am Rotationsverdampfer entfernt. Man erhält ein Rohprodukt, das man im Hochvakuum (1-10 Pa) destilliert. Anschliessend bestimmt man die chemische Ausbeute mittels Gaschromatographie (Kapillarsäule, OV-101). Die optische Ausbeute wird durch einen Vergleich mit dem $[\alpha]$ = -124,3° (C=3, Hexan) für das optisch reine sekundäre Amin ermittelt. Weitere Daten sind der folgenden Tabelle 1 entnehmbar.

Tabelle 1: Reaktion mit verschiedenen chiralen Diphosphinen (Ph=Phenyl)

| Chirales Diphosphin | Molverhältnis Substrat: Katalysator | Reaktionszeit (Stunden) | Chemische Ausbeute (%) | Optische Ausbeute (% ee) |
|---|---|---|---|---|
| 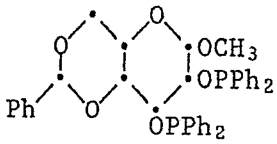 (α-POOP) | 50 | 192 | 73 | 7,8 |
| 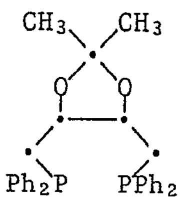 (DIOP) | 50 | 42 | 18 | 8,0 |
| 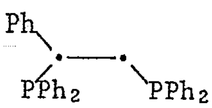 (PHENPHOS) | 50 | 18 | 83 | 21,2 |
| 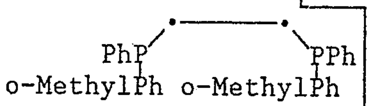 (DIPAMP) | 100 | 44 | 50 | 25,3 |
| 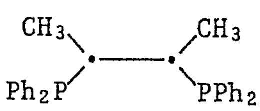 (CHIRAPHOS) | 50 | 66 | 88 | 37,8 |
| 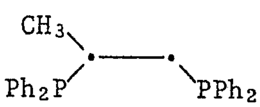 (PROPHOS) | 100 | 18 | 91 | 39,6 |
| 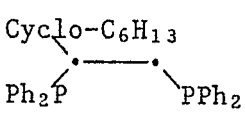 (CYCPHOS) | 100 | 44 | 99 | 52,8 |

Tabelle 1 (Fortsetzung)

| Chirales Diphosphin | Molverhältnis Substrat: Katalysator | Reaktionszeit (Stunden) | Chemische Ausbeute (%) | Optische Ausbeute (% ee) |
|---|---|---|---|---|
| 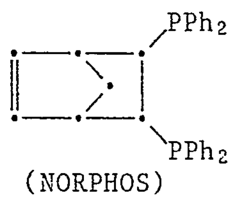 (NORPHOS) | 100 | 48 | 99 | 51,5 |

Reaktionsbedingungen

0,1 mMol Katalysator (hergestellt in situ aus 0,05 mMol $[Rh(Norbornadien)Cl]_2$ und 0,1 mMol chiralem Diphosphin bzw. Diphosphinit); Lösungmittel: 10 ml einer 1:1 Mischung von Methanol und Benzol; Temperatur 20°C, Wasserstoffdruck: $6{,}9 \cdot 10^6$ Pa.

Beispiel 2: Es wird wie in Beispiel 1 verfahren und die Reaktion bei verschiedenen Temperaturen durchgeführt. Das Verhältnis Substrat zu Katalysator beträgt 100. Der Druck beträgt $10{,}34.10^6$ Pa. Lösungsmittel bei den ersten beiden Reaktionen in Tabelle 2 ist 10 ml Methanol/Benzol(1:1), sonst 15 ml Methanol/Toluol(2:1).

Tabelle 2

| Chirales Diphosphin | Reaktionstemperatur (0°C) | Reaktionszeit (Stunden) | Chemische Ausbeute (%) | Optische Ausbeute (% ee) |
|---|---|---|---|---|
| NORPHOS | 5 | 68 | 100 | 59,5 |
| CYCPHOS | 4 | 66 | 100 | 60,2 |
| NORPHOS | - 10 | 68 | 100 | 64,0 |
| CYCPHOS | - 10 | 20 | 100 | 68,8 |
| CYCPHOS | - 25 | 70 | 100 | 73,0 |

Beispiel 3: Es wird wie im Beispiel 1 verfahren und CYCPHOS als chirales Diphosphin verwendet. Die Reaktion wird in 15 ml Methanol/Toluol (2:1) bei - 10°C und einem Wasserstoffdruck von $10{,}34 \cdot 10^6$ Pa mit unterschiedlichen Verhältnissen Substrat:Katalysator durchgeführt. Die Ergebnisse sind in Tabelle 3 angegeben.

Tabelle 3:

| Molverhältnis Substrat:Katalysator | Reaktions-zeit (Stunden) | Chemische Ausbeute (%) | Optische Aus-beute (% ee) |
|---|---|---|---|
| 1 : 200 | 24 | 31 | 68,8 |
| 1 : 500 | 66 | 81 | 68,2 |
| 1 : 1000 | 168 | 67 | 69,2 |

**Patentansprüche**

1. Verfahren zur Herstellung von optisch aktiven sekundären N-Arylaminen der Formel I

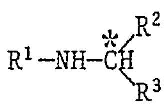
(I),

worin $R^1$ $C_6$-$C_{12}$-Aryl oder über ein Ring-C-Atom gebundenes $C_4$-$C_{11}$-Heteroaryl mit 1 oder 2 Heteroatomen im Ring bedeutet, die durch $C_1$-$C_{12}$-Alkyl, $C_1$-$C_{12}$-Alkoxy, $C_1$-$C_{12}$-Alkylthio, $C_1$-$C_6$-Haloalkyl, Halogen, -OH, -CN, $C_6$-$C_{12}$-Aryl oder -Aryloxy oder -Arylthio, $C_7$-$C_{16}$-Aralkyl oder -Aralkoxy oder -Aralkylthio, wobei die Arylreste ihrerseits durch $C_1$-$C_4$-Alkyl, -Alkoxy, -Alkylthio, Halogen, -OH, -CN, $-CONR^4R^5$ oder $-COOR^4$ substituiert sein können, Sekundäramino mit 2 bis 24 C-Atomen,

$-\overset{O}{\overset{\|}{C}}-NR^4R^5$ oder $-COOR^4$, wobei $R^4$ und $R^5$ unabhängig voneinander $C_1$-$C_{12}$-Alkyl, Phenyl oder Benzyl oder $R^4$ und $R^5$ zusammen Tetra- oder Pentamethylen oder 3-Oxapentylen sind, substituiert sein können,

$R^2$ und $R^3$ voneinander verschieden sind und gegebenenfalls durch -OH, -CN, Halogen, $C_1$-$C_{12}$-Alkoxy, Phenoxy, Benzyloxy, Sekundäramino mit 2 bis 24 C-Atomen,

$-\overset{O}{\overset{\|}{C}}NR^4R^5$ oder $-COOR^4$ substituiertes $C_1$-$C_{12}$-Alkyl oder Cycloalkyl mit 3-8 Ring-C-Atomen, gegebenenfalls wie $R^1$ substituiertes $C_6$-$C_{12}$-Aryl oder $C_7$-$C_{16}$-Aralkyl, $-CONR^4R^5$ oder $-COOR^4$ darstellen, worin $R^4$ und $R^5$ die zuvor angegebene Bedeutung haben; oder

$R^1$ die zuvor angegbene Bedeutung hat und $R^2$ und $R^3$ zusammen gegebenenfalls durch 1 oder 2 -O-, -S- oder $-NR^4$- unterbrochenes, und/oder gegebenenfalls durch =O oder wie zuvor für $R^2$ und $R^3$ in der Bedeutung von Alkyl substituiertes, und/oder mit Benzol, Furan, Thiophen oder Pyrrol kondensiertes Alkylen mit 2 bis 5 C-Atomen sind, oder $R^2$ die zuvor angegebene Bedeutung hat und $R^3$ an $R^1$ gebundenes, gegebenenfalls durch 1 oder 2 -O-, -S- oder $-NR^4$ - unterbrochenes, und/oder gegebenenfalls durch =O oder wie zuvor für $R^2$ und $R^3$ in der Bedeutung von Alkyl substituiertes, und/oder mit Benzol, Furan, Thiophen oder Pyrrol kondensiertes Alkylen mit 2 bis 5 C-Atomen sind, und * für überwiegend R- oder S-Konfiguration steht, durch asymmetrisch katalysierte Hydrierung von N-arylierten prochiralen Ketiminen der Formel II

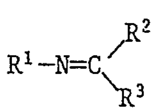
(II),

worin $R^1$, $R^2$ und $R^3$ die zuvor angegebenen Bedeutungen haben, in Gegenwart von Komplexsalzen eines Edelmetalls mit chiralen Liganden, dadurch gekennzeichnet, dass man die Hydrierung bei einer Temperatur von -40 bis 80°C und einem Wasserstoffdruck von $10^6$ Pa bis $10^8$ Pa vornimmt und dem Reaktionsgemisch katalytische Mengen einer Rhodiumverbindung der Formel III oder IIIa

$[XRhYZ]$ (III) oder $[XRhY]^{\oplus}A^{\ominus}$ (IIIa)

zugibt, worin X für zwei Olefinliganden oder einen Dienliganden steht, Y ein chirales Diphosphin, dessen sekundäre Phosphingruppen durch 2-4 C-Atome verknüpft sind und das zusammen mit dem Rh-Atom einen 5-, 6- oder 7-Ring bildet, oder Y ein chirales Diphosphinit, dessen Phosphinitgruppen über 2 C-Atome verknüpft sind und das zusammen mit dem Rh-Atom einen 7-Ring bildet, bedeutet, Z für Cl, Br oder I steht, und $A^{\ominus}$ das Anion einer Sauerstoff- oder Komplexsäure darstellt.

2. Verfahren gemäss Anspruch 1, dadurch gekennzeichnet, dass die Reaktionstemperatur -30 bis 50°C beträgt.

3. Verfahren gemäss Anspruch 1, dadurch gekennzeichnet, dass der Wasserstoffdruck $4 \cdot 10^6$ Pa bis $5 \cdot 10^7$ Pa beträgt.

4. Verfahren gemäss Anspruch 1, dadurch gekennzeichnet, dass X in den Formeln III und IIIa zwei Ethylen oder ein offenkettiges oder cyclisches Dien ist, dessen Diengruppen über 1 oder 2 C-Atome verknüpft sind.

5. Verfahren gemäss Anspruch 4, dadurch gekennzeichnet, dass das Dien Hexadien, Norbornadien oder Cyclooctadien ist.

6. Verfahren gemäss Anspruch 1, dadurch gekennzeichnet, dass Y in den Formeln III und IIIa ein chirales Diphosphin ist, dessen Phosphingruppen durch 2 C-Atome verknüpft sind und das zusammen mit dem Rh-Atom einen 5-Ring bildet.

7. Verfahren gemäss Anspruch 1, dadurch gekennzeichnet, dass die Phosphingruppen $C_1$-$C_{12}$-Alkyl, Cycloalkyl mit 5 bis 8 Ring-C-Atomen, das durch 1 bis 3 $C_1$-$C_6$-Alkylgruppen substituiert sein kann, Phenyl, $C_7$-$C_{12}$-Phenylalkyl oder Alkylphenylalkyl mit 1 bis 6 C-Atomen in den Alkylgruppen und 1 bis 5 C-Atomen in der Alkylengruppe enthalten.

8. Verfahren gemäss Anspruch 1, dadurch gekennzeichnet, dass $A^{\ominus}$ für $ClO_4^{\ominus}$, $CF_3SO_3^{\ominus}$, $BF_4^{\ominus}$, $PF_6^{\ominus}$, $SbCl_6^{\ominus}$, AsFs

10. Verfahren gemäss Anspruch 1, dadurch gekennzeichnet, dass in Formel III X für Cyclooctadien, Z für Cl und Y für (R)- oder (S)-

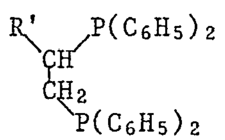

stehen, worin R′ Methyl, Phenyl oder Cyclohexyl bedeutet.

11. Verfahren gemäss Anspruch 1, dadurch gekennzeichnet, dass in Formel II $R^1$ für 2,6-Dimethylphen-1-yl oder 2-Methyl-6-ethylphen1-yl, $R^2$ für Methyl und $R^3$ für Methoxymethyl stehen.

12. Verfahren gemäss Anspruch 1, dadurch gekennzeichnet, dass es sich bei dem Diphosphinit um 1-0-Phenyl-4,6-0-(R)-benzyliden-2,3-0-bis(diphenylphosphino)-β-D-glucopyranosid handelt.